# EUROPÄISCHE PATENTANMELDUNG

(11) **EP 1 371 624 A1**
(43) Veröffentlichungstag der Anmeldung: **17.12.2003**
(21) Anmeldenummer: 03009111.0
(22) Anmeldetag: 19.04.2003
(51) Int. Cl.: C07C 41/60, C07C 43/32

(54) **Verfahren zur Herstellung von 2-Chloro-1,1,1-trialkoxyethan**

(30) Priorität: 10.06.2002 DE 10225750
(71) Anmelder: Degussa AG, 40474 Düsseldorf (DE)
(72) Erfinder: Köhler, Günther, Dr., 45770 Marl (DE); Brühl, Karl-Heinz, 46242 Velen (DE); Ruwwe, Johannes, Dr., 53859 Niederkassel (DE); Neumann, Manfred, Dr., 45770 Marl (DE)

(57) **Zusammenfassung**

Die Erfindung betrifft ein Verfahren zur Herstellung von 2-Chloro-1,1,1-trialkoxyethan (II) (monochlorierte Essigsäureorthoester) durch Umsetzung von 1,1,1-Trialkoxyethan (I) (Orthoessigsäureester) mit Chlor vorzugsweise in Gegenwart von Alkoholen und katalytischen Mengen einer Base.

## Beschreibung

Die Erfindung betrifft ein Verfahren zur Herstellung von 2-Chloro-1,1,1-trialkoxyethan (II) (monochlorierte Essigsäureorthoester) durch Umsetzung von 1,1,1-Trialkoxyethan (I) (Orthoessigsäureester) mit Chlor vorzugsweise in Gegenwart von Alkoholen und vorzugsweise in Gegenwart von katalytischen Mengen einer Base: 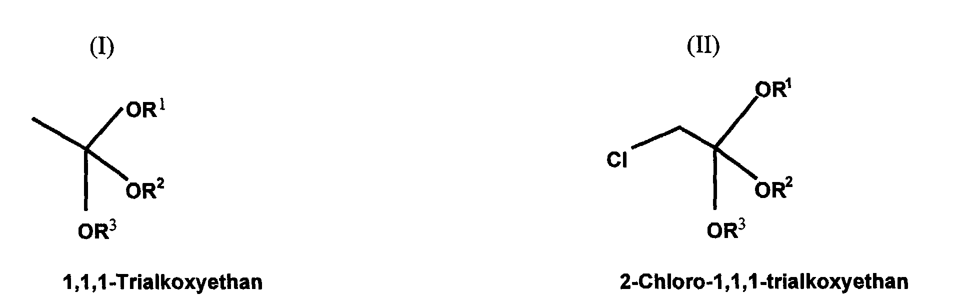 2-Chloro-1,1,1-trialkoxyethane (chlorierte Orthoessigsäureester) sind wichtige Rohstoffe und Bausteine für Wirkstoffe in der Pharma- oder Agroindustrie. Insgesamt sind sie vielseitig zum Aufbau von Heterozyklen geeignet.

So wird die Anwendung von chlorierten Orthoessigsäureestern in Tetrahedron Letters, 41(44), 8661 - 8664; 2000 und in der Patentanmeldung WO01/96 315 als Edukt bei der Umsetzung mit Semicarbazidhydrochlorid zu substituierten Triazolonen oder in Journal of Organic Chemistry, 66 (18) 6116 - 6123, 2001 und in Tetrahedron Letters 41 (39), 7447 - 7452, 2000 als Baustein für substituierte cyclische Ketale oder Lactone beschrieben.

In der Patentschrift US 5 831 089 dient 2-Chloro- 1,1,1 -trialkoxyethan als Baustein mit einem Amin als Reaktand zur Herstellung von substituierten Säureamidoestern.
In Synth. Commun. 19 (16), 2921 - 2924 wird sowohl die Anwendung von 2-Chloro-1,1,1-triethoxyethan als vielseitiger Baustein zur Herstellung von Benzothiazolen und Benzoxazolen als auch die Herstellung durch Umsetzung von Triethylorthoacetat mit N-Chlorsuccinimid beschrieben.
Die Herstellung von 2-Chloro-1,1,1-orthoestem wird nach dem Stand der Technik durch Umsetzung von Orthoestern mit Chlorsuccinimid vielfach in großen Mengen chlorierter Kohlenwasserstoffe ausgeführt. So wird in der europäischen Patentanmeldung EP 0 222 576 die Synthese von 2-Chloro-1,1,1-triethoxyethan ausgehend von 1,1,1-Triethoxyethan mit N-Chlorsuccinimid in Gegenwart von Tetrachlorkohlenstoff als Lösemittel beschrieben. Weitere Beispiele nach dem Stand der Technik sind in den Patentschriften EP 0 222 576 und US 4 748 280 (31.7.1987) zu finden, wobei 2-Chloro- 1,1,1 -(C1-C4)-alkoxyethan durch Umsetzung von 1,1,1-(C1-C4)-alkoxyethan mit N-Chlorsuccinimid in Gegenwart von chlorierten Kohlenwasserstoffen als Lösemittel synthetisiert wird. In der zuletzt zitierten Patentschrift wird ein weiterer Syntheseweg vorgeschlagen, wobei das gleiche Edukt mit Chlor in Gegenwart von stöchiometrischen Mengen Pyridin als Base umgesetzt wird, wobei Chlorkohlenwasserstoff als Cosolvens dient.

Allen diesen Verfahren ist gemeinsam, dass die Raum-Zeit-Ausbeute unter dem Aspekt einer technischen und industriellen Anwendung nicht wirtschaftlich ist und die Verfahren nur mit sehr hohem Aufwand ausführbar sind. Zudem werden häufig Halogenkohlenwasserstoffe als Lösemittel eingesetzt, die unter Umweltschutzaspekten in der chemischen Industrie ungern Anwendung finden. Ein weiterer wirtschaftlicher Mangel der herkömmlichen Verfahren bei Verwendung von N-Chlorsuccinimid ist der sehr hohe Preis dieses Rohstoffes. Darüber hinaus fällt bei der Verwendung von N-Chlorsuccinimid nach erfolgter Synthese Succinimid als Abfall an, das als Feststoff sehr aufwändig aus dem Reaktionsgemisch über eine Feststoffaufarbeitung (Filter, Dekanter oder Zentrifugen) abgetrennt werden muss. Auch das macht eine technische Anwendung sehr nachteilig. Neben den bereits erwähnten Mängeln ist allen Verfahren nach dem Stand der Technik gemeinsam, dass ein relativ hoher Anteil 2,2-Dichloro-1,1,1-trialkoxyethan anfällt, das bei der anschließenden Aufarbeitung des Reaktionsgemisches nur mit sehr hohem destillativen Aufwand von Monochloro-1,1,1-trialkoxyethan abtrennbar ist. Für Anwendungen im Pharmabereich ist es jedoch wünschenswert, ein sehr reines Produkt mit einem sehr geringen Dichloranteil als Nebenprodukt zu erhalten.

Es wurde nun überraschend gefunden, dass man 2-Chloro-1,1,1-trialkoxyethan mit hoher Reinheit herstellen kann, wenn man 1,1,1-Trialkoxyethan mit gasförmigem oder flüssigem Chlor umsetzt, wobei die Reaktion vorzugsweise in Gegenwart eines Alkohols und vorzugsweise einer katalytischen Menge an Base durchgeführt wird.

Die Alkoxyreste R₁, R₂ und R₃ sind gleich oder verschieden und enthalten 1 bis 8, vorzugsweise 1 bis 6 und besonders bevorzugt 1 bis 4 Kohlenstoffatome im Alkylrest. Sie können aber auch über eine Alkylenbrücke miteinander verbunden sein. Als Alkylreste seien insbesondere der Methyl-, Propyl-, Isopropyl, n-Butyl, Isobutyl und sec.-Butylrest genannt. Im Falle einer Alkylenbrücke enthält diese vorzugsweise 2 - 3 Kohlenstoffatome.
Der Alkohol kann linear oder verzweigt sein oder auch einen aromatischen Rest enthalten. Die Gesamtzahl der Kohlenstoffatome im Alkohol beträgt 1 bis 10. Als Beispiele seien Methanol, Ethanol, die isomeren Propanole, die isomeren Butanole und Benzylalkohol genannt.

Das erfindungsgemäße Verfahren wird bei - 20 °C bis + 100 °C und vorzugsweise bei Normaldruck ausgeführt. Geringe Überdrucke bis etwa 10 bar, vorzugsweise 5 bar (absolut) sollen aber nicht ausgeschlossen werden. Es finden relativ geringe Mengen Lösemittel Anwendung, wobei niedrigsiedende und destillativ leicht abtrennbare niedermolekulare Alkohole bevorzugt werden. Der Anteil an Alkoholen liegt im Allgemeinen bei 0,1 bis 20 %, vorzugsweise 5 bis 20 %, bezogen auf das eingesetzte 1,1,1-Trialkoxyethan. Die Reaktion kann auch gänzlich ohne einen Alkohol gestartet werden, wobei in diesem Fall die destillative Aufarbeitung zur Gewinnung des reinen Reaktionsproduktes sogar einfacher ist. Ohne die Zugabe von Alkoholen ist allerdings mit einem größeren Anteil an Nebenprodukten zu rechnen, wobei vor allem Essigsäureester durch den Abbau der eingesetzten Orthoacetate festgestellt wurden.
Zur Chlorierung wird gasförmiges oder flüssiges Chlor eingesetzt, das in den Reaktor beziehungsweise in das Orthoacetat oder die vorgelegte Mischung Orthoacetat/Alkohol dosiert wird. Weil die Chlorierung stark exotherm ist (ΔH ca. - 100 kJ/mol), wird Chlor vorzugsweise absatzweise oder langsam mit einer geringen Dosierrate hinzugefahren, so dass die Innentemperatur nicht so weit ansteigt, dass die Selektivität negativ beeinflusst wird. Es wurde festgestellt, dass ein tolerabel geringes Nebenproduktspektrum innerhalb eines Temperaturbereiches von - 20 °C bis maximal 100 °C auftrat.

Der Einsatz von katalytischen Mengen einer Base beim Start der Chlorierung erwies sich überraschenderweise als vorteilhaft. Als Base eignen sich geringe Mengen Alkalihydroxid oder Alkalialkoholat, insbesondere Natrium- oder Kaliumalkoholat wie zum Beispiel Natriummethylat, Natriumethylat, Natriumisopropylat, Kaliummethylat, Kaliumethylat, Kalium-tert.butylat oder Kalium-sec.butylat. Die Basen können als Reinstoff zum Beispiel als Feststoff in Pulver- oder Granulat-Form oder aber auch als alkoholische Lösung eingesetzt werden. Die Mengen an reiner Base sollen, wenn verwendet, erfindungsgemäß zwischen 0,1 und 10 %, vorzugsweise zwischen 0,2 und 1 % (bezogen auf eingesetztes Trialkoxyethan), liegen.

Der Gehalt an Alkohol, der zur Lösung der Base eingesetzt werden kann, sollte vorzugsweise aus wirtschaftlichen Gesichtspunkten nicht größer als 20 % bezogen auf den eingesetzten Orthoester betragen.

Das Verfahren kann absatzweise oder auch kontinuierlich betrieben werden. Im Falle der kontinuierlichen Verfahrensdurchführung werden der Reaktionsmischung nur so viele Teile entnommen, wie an Edukten hinzugefügt werden, so dass der Füllstand im Reaktor konstant bleibt. Bei Start der Reaktion ist es ratsam, den Umsatz auf ein sehr hohes Niveau zu bringen. Die erforderliche Reaktions- oder Verweilzeiten liegen in Abhängigkeit von der abführbaren Reaktionswärme in einem Bereich von 2 - 10 Stunden. Die Zeiten werden im Wesentlichen durch die verfügbare Kälteleistung und die Reaktorausführung bestimmt.
Alle erwähnten Verfahrensvarianten bringen jedoch gegenüber herkömmlichen Verfahren nach dem Stand der Technik einen besseren Umsatz innerhalb kurzer Reaktionszeiten bei sehr hoher Selektivität. Der Dichloranteil des Orthoesters liegt in Abhängigkeit vom Orthoester oder dem eingesetzten Alkoholanteil meist weit unter zwei Prozent, so dass die destillative Reinigung unter wirtschaftlich tolerablen Destillationsverlusten zu einem hochreinen Monochlororthoacetat führt.
Im Unterschied zu herkömmlichen Verfahren fallen keine Feststoffe oder - mit Ausnahme bei Verwendung von geringen Mengen Alkalialkoholat - in Form von Chloriden an. Derart geringe Mengen Feststoff von ca. 0,1 bis 1 % lassen sich jedoch leicht mechanisch zum Beispiel mit einfachen Filterkerzen abtrennen, bevor die Reaktionsmischung destillativ aufgearbeitet wird, wodurch Feststoffablagerungen in den Destillationsanlagen vermieden werden.

### Beispiel 1:

In einen 500 ml Kolben mit Rührer und Gaseinleitungsrohr wurde bei 10 °C eine Mischung von 240 g (2 mol) 1,1,1-Trimethoxyethan und 1 % NaOCH₃ (30 %ig) vorgelegt. Innerhalb von 4 Stunden wurden 110 g Chlor gasförmig eingeleitet, wobei die Innentemperatur nicht über 15 °C steigt. Nachdem die gesamte Chlormenge eingeleitet worden war, wurde die Mischung destillativ aufgearbeitet. Über eine Destillationsapparatur mit ca. 5 theoretischen Trennstufen wurden im Vorlauf Leichtsieder wie Methanol und Methylacetat bei einem Rücklaufverhältnis von 5 : 1 abgetrennt. Im Hauptlauf wurden bei einem Rücklaufverhältnis von 2 : 1 234 g 2-Chloro-1,1,1-trimethoxyethan mit einem Gehalt > 99,0 % erhalten. Dies entspricht einer Ausbeute von ca. 76 %.

### Beispiel 2:

In einen 1000 ml Kolben mit Rührer und Gaseinleitungsrohr wurde bei 10 °C eine Mischung von 480 g (4 mol) 1,1,1-Trimethoxyethan und 48 g (10 Gew %) Methanol vorgelegt. Innerhalb von 4 Stunden wurden 220 g (3,14 mol) Chlor gasförmig eingeleitet, wobei die Innentemperatur nicht über 15 °C steigt. Nach dem die gesamte Chlormenge eingeleitet worden ist, wurde die Mischung destillativ aufgearbeitet. Über eine Destillationsapparatur mit ca. 5 theoretischen Trennstufen wurden im Vorlauf Leichtsieder wie Methanol und Methylacetat bei einem Rücklaufverhältnis von 5 : 1 abgetrennt. Im Hauptlauf wurden bei einem Rücklaufverhältnis von 2 : 1 425 g 2-Chloro-1,1,1-trimethoxyethan mit einem Gehalt > 99,0 % erhalten. Dies entspricht einer Ausbeute von ca. 69 %.

### Beispiel 3:

In einen 1000 ml Kolben mit Rührer und Gaseinleitungsrohr wurden bei 10 °C 480 g (4 mol) 1,1,1-Trimethoxyethan vorgelegt. Innerhalb von 4 Stunden wurden 220 g (3,14 mol) Chlor gasförmig eingeleitet, wobei die Innentemperatur nicht über 15 °C stieg. Nachdem die gesamte Chlormenge eingeleitet worden war, wurde die Mischung destillativ aufgearbeitet. Über eine Destillationsapparatur mit ca. 5 theoretischen Trennstufen wurden im Vorlauf Leichtsieder wie Methanol und Methylacetat bei einem Rücklaufverhältnis von 5 : 1 abgetrennt. Im Hauptlauf wurden bei einem Rücklaufverhältnis von 2 : 1 413 g 2-Chloro-1,1,1-trimethoxyethan mit einem Gehalt > 99,0 % erhalten. Dies entspricht einer Ausbeute von ca. 67 %.

### Beispiel 4:

In einen 1000 ml Kolben mit Rührer und Gaseinleitungsrohr wurde bei 10 °C eine Mischung von 648 g (4 mol) 1,1,1-Triethoxyethan und 70 g (ca. 11 Gew.- %) Ethanol vorgelegt. Innerhalb von 4 Stunden wurden 260 g (3,70 mol) Chlor gasförmig eingeleitet, wobei die Innentemperatur nicht über 15 °C stieg. Nachdem die gesamte Chlormenge eingeleitet worden war, wurde die Mischung destillativ aufgearbeitet. Über eine Destillationsapparatur mit ca. 5 theoretischen Trennstufen wurden im Vorlauf Leichtsieder wie Methanol und Methylacetat bei einem Rücklaufverhältnis von 5 : 1 abgetrennt. Im Hauptlauf wurden bei einem Rücklaufverhältnis von 2 : 1 520 g 2-Chloro-1,1,1-trimethoxyethan mit einem Gehalt > 99,0 % erhalten. Dies entspricht einer Ausbeute von ca. 67 %.

## Patentansprüche

1. Verfahren zur Herstellung von 2-Chloro-1,1,1-trialkoxyethan durch Umsetzung von 1,1,1-Trialkoxyethan mit gasförmigem oder flüssigem Chlor, wobei die Alkoxyreste gleich oder verschieden oder über eine Alkylenbrücke verbunden sein können, in Gegenwart von 0,1 % bis 20 % Alkohol.

2. Verfahren zur Herstellung von 2-Chloro-1,1,1-trialkoxyethan nach Anspruch 1,
**dadurch gekennzeichnet,**
**dass** die Alkoxyreste 1 bis 8 Kohlenstoffatome enthalten.

3. Verfahren zur Herstellung von 2-Chloro-1,1,1 -trialkoxyethan nach Anspruch 2,
**dadurch gekennzeichnet,**
**dass** die Alkyoxyreste eine Methyl-, Ethyl-, Propyl-, Isopropyl-, n-Butyl-, Isobutyl- oder sec. Butylgruppen tragen oder im Falle einer Alkylenbrücke die Brücke 2 - 3 Kohlenstoffe trägt.

4. Verfahren zur Herstellung von 2-Chloro-1,1,1 -trialkoxyethan nach Anspruch 1,
**dadurch gekennzeichnet,**
**dass** ein Alkohol mit 1 bis 10 Kohlenstoffatomen anwesend ist.

5. Verfahren zur Herstellung von 2-Chloro-1,1,1-trialkoxyethan nach Anspruch 4,
**dadurch gekennzeichnet,**
**dass** der Alkohol eine geradkettig oder verzweigte Kohlenstoffkette oder auch einen aromatischen Rest enthält.

6. Verfahren zur Herstellung von 2-Chloro-1,1,1-trialkoxyethan nach Anspruch 1,
**dadurch gekennzeichnet,**
**dass** die Alkoholmengen bezogen auf das eingesetzte 1,1,1-Trialkoxyethan 5 % bis 20 % betragen.

7. Verfahren zur Herstellung von 2-Chloro-1,1,1-trialkoxyethan nach einem der vorhergehenden Ansprüche,
**dadurch gekennzeichnet,**
**dass** eine Base zugesetzt wird.

8. Verfahren zur Herstellung von 2-Chloro-1,1,1-trialkoxyethan nach einem der vorhergehenden Ansprüche,
**dadurch gekennzeichnet,**
**dass** die Base ein Alkalialkoholat ist.

9. Verfahren zur Herstellung von 2-Chloro-1,1,1-trialkoxyethan nach einem der vorhergehenden Ansprüche,
**dadurch gekennzeichnet,**
**dass** die Mengen an Base 0,1 bis 10 % beträgt.

10. Verfahren zur Herstellung von 2-Chloro-1,1,1-trialkoxyethan nach einem der vorhergehenden Ansprüche,
**dadurch gekennzeichnet,**
**dass** die Temperatur bei der Reaktion - 20 °C bis 100 °C beträgt.

11. Verfahren zur Herstellung von 2-Chloro-1,1,1-trialkoxyethan nach einem der vorhergehenden Ansprüche,
**dadurch gekennzeichnet,**
**dass** die Reaktion bei 1 bis 5 bar absolut durchgeführt wird.

12. Verfahren zur Herstellung von 2-Chloro-1,1,1-trialkoxyethan nach einem der vorhergehenden Ansprüche,
**dadurch gekennzeichnet,**
**dass** die Reaktion absatzweise oder kontinuierlich durchgeführt wird.
